# EUROPÄISCHE PATENTSCHRIFT

(11) **EP 3 639 779 B1**
(45) Veröffentlichungstag und Bekanntmachung des Hinweises auf die Patenterteilung: **10.08.2022**
(21) Anmeldenummer: 18200797.1
(22) Anmeldetag: 16.10.2018
(51) Int. Cl.: A61B 18/14, A61B 18/12

(54) **INSTRUMENT ZUR KOAGULATION UND DISSEKTION VON BIOLOGISCHEM GEWEBE**
INSTRUMENT FOR COAGULATION AND DISSECTION OF BIOLOGICAL TISSUE
INSTRUMENT DE COAGULATION ET DE DISSECTION DE TISSU BIOLOGIQUE

(43) Veröffentlichungstag der Anmeldung: 22.04.2020
(73) Patentinhaber: Erbe Elektromedizin GmbH, 72072 Tübingen (DE)
(72) Erfinder: RIPPLINGER, Thomas, 72072 Tübingen (DE)
(74) Vertreter: Rüger Abel Patentanwälte PartGmbB

(56) Entgegenhaltungen:
- WO-A1-02/100283
- DE-A1- 4 332 614
- US-A1- 2014 018 795

## Beschreibung

Die Erfindung betrifft ein Instrument zur Koagulation und Dissektion von biologischem Gewebe.

Ein solches Instrument ist beispielsweise aus EP 3 132 765 A1 bekannt. Das Instrument hat ein Werkzeug mit einer Schneidelektrode und mehreren Koagulationselektroden. Mittels einer Betriebsschaltung können die Elektroden mit elektrischer Energie versorgt werden. Hierzu weist die Betriebsschaltung einen Transformator auf, der sekundärseitig über einen Leistungsschalter mit der Schneidelektrode verbunden ist. Das Instrument hat einen Schneidaktivierungsschalter und einen Koagulationsaktivierungsschalter. Der Koagulationsaktivierungsschalter ist mechanisch mit dem Leistungsschalter gekoppelt. Beim Betätigen des Koagulationsaktivierungsschalters wird die elektrische Verbindung zwischen der Schneidelektrode und dem Transformator getrennt oder umgeschaltet, so dass keine Schneidspannung an der Schneidelektrode anliegt.

WO 02/100283 A1 beschreibt eine elektrochirurgische Vorrichtung mit einem Generator und einem Instrument. Das Instrument hat einen ersten Schalter, bei dessen Betätigung das Instrument zum Koagulieren verwendet werden kann, sowie einen zweiten Schalter, bei dessen Betätigung das Instrument zum Schneiden verwendet werden kann. Den beiden Schaltern sind Signalkodiermittel zugeordnet, um abhängig davon, welche Schalter betätigt wurde, ein Steuersignal zu erzeugen, das über eine Steuersignalleitung dem Generator übermittelt wird. Der Generator wertet das Steuersignal aus und aktiviert entweder den Koagulations- oder Schneidmodus für das angeschlossene Instrument.

US 2014/0018795 A1 offenbart ebenfalls ein Instrument mit mehreren Schaltern, mittels dem ein Betriebsmodus zum Koagulieren oder ein Betriebsmodus zum Schneiden eines Generators aktiviert werden kann, an den das Instrument angeschlossen ist.

DE 43 32 614 A1 beschreibt ein Röntgengerät, das derart ausgestaltet ist, dass eine Bedienperson beide Hände oder eine Hand und einen Fuß zur Steuerung des Röntgengeräts verwenden muss. Dadurch wird vermieden, dass unbeabsichtigt durch Betätigen eines einzigen Knopfes eine ungewollte Steuerung des Röntgengeräts erfolgt.

Instrumente zur Koagulation bzw. Dissektion von biologischem Gewebe werden sowohl als Mehrweginstrument als auch als Einweginstrument angeboten. Insbesondere bei Einweginstrumenten spielen die Herstellungskosten eine große Rolle. Es kann daher als Aufgabe der vorliegenden Erfindung angesehen werden, ein Instrument zu schaffen, das einfach und sicher zu bedienen ist und sich kostengünstig herstellen lässt.

Diese Aufgabe wird durch ein Instrument mit den Merkmalen des Patentanspruches 1 gelöst.

Das Instrument ist zur Koagulation und Dissektion von biologischem Gewebe eingerichtet. Es hat ein Werkzeug mit wenigstens einer Schneidelektrode und wenigstens einem Koagulationselektrodenpaar. Jedes Koagulationselektrodenpaar hat eine erste Koagulationselektrode und eine zweite Koagulationselektrode. Das Instrument weist eine Betriebsschaltung auf. Die Betriebsschaltung hat einen mit der Schneidelektrode verbundenen Schneidausgang, einen mit der wenigstens einen ersten Koagulationselektrode verbundenen ersten Koagulationsausgang und einen mit der wenigstens einen zweiten Koagulationselektrode verbundenen zweiten Koagulationsausgang. Die Betriebsschaltung weist außerdem einen Versorgungsanschluss auf, mittels dem eine Versorgungsspannung oder ein Versorgungsstrom bereitgestellt wird. Ferner ist ein Auswerteanschluss vorhanden, mittels dem ein periodisches Auswertesignal mit ersten Halbwellen einer Polarität und zweiten Halbwellen der jeweils anderen Polarität bereitgestellt wird. Beispielsweise sind die ersten Halbwellen negative Halbwellen und die zweiten Halbwellen positive Halbwellen des Auswertesignals. Das Auswertesignal kann ein Strom oder eine Spannung sein.

Die Betriebsschaltung hat außerdem eine mit dem Auswerteanschluss verbundene Auswerteschaltung aufweisend einen manuell betätigbaren ersten Schalter und ein Ansteuerelement zur Erzeugung eines Ansteuersignals. Das Ansteuerelement kann ein einzelnes Bauelement sein oder eine Gruppe aus mehreren Einzelelementen. Die Auswerteschaltung ist dazu eingerichtet, während wenigstens einer der ersten Halbwellen auszuwerten, ob der erste Schalter betätigt wurde. Abhängig von diesem Auswerteergebnis wird dann beginnend mit einer der nachfolgenden zweiten Halbwellen das dem Auswerteergebnis entsprechende Ansteuersignal erzeugt. Mittels des Ansteuersignals wird eine ansteuerbare Umschalteinheit zwischen einem ersten Schaltzustand und einem zweiten Schaltzustand umgeschaltet. Die Umschalteinheit kann mittelbar oder unmittelbar mit dem Versorgungseingang bzw. dem Schneidausgang verbunden sein. Sie kann bei einem bevorzugten Ausführungsbeispiel in Reihe zwischen dem Versorgungsanschluss und dem Schneidausgang angeordnet sein. In diesem Fall kann die Umschalteinheit beispielsweise zwischen einem leitenden und einem sperrenden Schaltzustand umgeschaltet werden. Es ist auch möglich, die Umschalteinheit zwischen zwei leitenden Schaltzuständen umzuschalten, wobei der Schneidausgang in diesem Fall mit unterschiedlichen Spannungen oder Strömen versorgt wird. Im ersten Schaltzustand der Umschalteinheit liegt am Schneidausgang und mithin an der Schneidelektrode eine elektrische Spannung an oder es wird ein elektrischer Strom bereitgestellt, der zur Dissektion von biologischem Gewebe geeignet ist. Demgegenüber ist an der Schneidelektrode im zweiten Schaltzustand keine elektrische Spannung oder kein elektrischer Strom verfügbar, der ausreichend ist für die Dissektion von biologischem Gewebe. Im zweiten Schaltzustand kann entweder im Wesentlichen keine Spannung oder kein Strom an der Schneidelektrode verfügbar sein oder alternativ eine Spannung oder ein Strom bereitgestellt werden, der für die Dissektion nicht ausreichend ist, aber z.B. für die Koagulation geeignet ist.

Das Auswertesignal wird zur Abfrage, ob der erste Schalter betätigt wurde, durch ein Gerät bereitgestellt, an das das Instrument anschließbar ist. Der Betätigungszustand des ersten Schalters wird während wenigstens einer ersten Halbwelle ausgewertet und bei einer Änderung des Betätigungszustands wird während wenigstens einer der darauf folgenden zweiten Halbwellen das Ansteuersignal an dem geänderten Betätigungszustand des ersten Schalters angepasst. Außerdem kann das Gerät dem Instrument am Versorgungseingang bei festgestellter Betätigung des ersten Schalters eine Versorgungsspannung oder einen Versorgungsstrom bereitstellen. Insbesondere wird die Umschalteinheit bei unbetätigtem ersten Schalter durch das Ansteuersignal in den zweiten Schaltzustand umgeschaltet, in dem keine elektrische Energie für die Dissektion an der Schneidelektrode bereitsteht. Wird das Betätigen des ersten Schalters erkannt, wird ein Ansteuersignal erzeugt, das die Umschalteinheit in den ersten Schaltzustand umschaltet, in dem dann an der Schneidelektrode eine elektrische Energie für die Dissektion bereitgestellt wird.

Eine mechanische Kopplung zwischen der Umschalteinrichtung und der Auswerteschaltung kann entfallen. Das Instrument lässt sich sehr einfach mit Standard-Bauelementen aufbauen. Insbesondere kann die Umschalteinheit über einen oder mehrere Halbleiterschalter aufgebaut werden. Mechanische Schalter können in der Umschalteinheit entfallen. Dadurch ist eine einfache Vermeidung von Funken- oder Lichtbogenbildung beim Ändern des Schaltzustands erreicht. Die Lösung kann kostengünstig auch bei Einweginstrumenten realisiert werden.

Es ist vorteilhaft, wenn die Betriebsschaltung eine Kopplungseinrichtung aufweist, die ein Senderbauteil und ein galvanisch vom Senderbauteil getrenntes Empfängerbauteil aufweist. Über das Senderbauteil kann das Ansteuersignal von der Auswerteschaltung zum Empfängerbauteil übertragen werden, das mit der Umschalteinheit verbunden ist oder zur Umschalteinheit gehört.

Bei einem Ausführungsbeispiel ist das Senderbauteil durch das Ansteuerelement gebildet. Dabei kann das Empfängerbauteil mit einem Steuereingang eines ansteuerbaren Halbleiterschalters der Umschalteinrichtung verbunden sein.

Als Senderbauteil kann beispielsweise wenigstens eine Leuchtdiode und als Empfängerbauteil wenigstens eine Fotodiode oder wenigstens eine Fototransistor verwendet werden. Beispielsweise kann das Senderbauteil und das Empfängerbauteil als gemeinsame Baugruppe in einem gemeinsamen Gehäuse angeordnet werden. Diese Baugruppe kann beispielsweise ein Optokoppler sein.

Es ist vorteilhaft, wenn das Empfängerbauteil über eine Lade- und Entladeschaltung mit dem wenigstens einen Steuereingang der Umschalteinheit verbunden ist. Mittels der Lade- und Entladeschaltung ist es möglich den ersten Schaltzustand der Umschalteinheit bei einer Dissektionsanforderung (z.B. Betätigen des ersten Schalters) auch während der Zeitdauer aufrechtzuerhalten, während der eine erste Halbwelle anliegt. Vorzugsweise ist die Lade- und Entladeschaltung auch dazu eingerichtet, die elektrischen Ladungen im wenigstens einen Steuereingang der Umschalteinheit abzubauen, wenn keine zur Dissektion geeignete Spannung an die Schneidelektrode angelegt werden soll, um ein Umschalten der Umschalteinheit in den zweiten Schaltzustand zu ermöglichen.

Die Umschalteinheit kann bei einem Ausführungsbeispiel ohne mechanische Schalter ausgebildet sein und ausschließlich Halbleiterschalter aufweisen, wie etwa Bipolartransistoren und/oder Feldeffekttransistoren und/oder IGBTs.

Es ist vorteilhaft, wenn der erste Schalter und das Ansteuerelement in einem ersten Auswertezweig in Reihe geschaltet sind. Der erste Auswertezweig kann mit dem Auswerteanschluss verbunden sein. Dadurch kann ein Stromfluss durch das Ansteuerelement bei geöffnetem ersten Schalter unterbunden werden.

Beispielsweise kann im ersten Auswertezweig ein Einbahnstrompfad vorhanden sein, in dem das Ansteuerelement angeordnet ist. In dem Einbahnstrompfad wird der Stromfluss nur in eine Richtung zugelassen, insbesondere in die Stromflussrichtung bei anliegender zweiter Halbwelle des Auswertesignals. In dem Einbahnstrompfad ist dazu wenigstens ein Bauteil mit Diodenfunktion vorhanden. Das Bauteil mit Diodenfunktion kann beispielsweise das Ansteuerelement selbst sein. Alternativ oder zusätzlich kann ein weiteres Bauteil mit Diodenfunktion, im einfachsten Fall eine Diode, im Einbahnstrompfad vorhanden sein. Alternativ zur Diode können auch gesteuerte Halbleitschalter verwendet werden, die nur bei einer zweiten Halbwelle des Auswertesignals ihren leitenden Zustand einnehmen.

Bei einem Ausführungsbeispiel ist das Ansteuerelement als Leuchtdiode ausgeführt. Zusätzlich zu der Leuchtdiode kann im Einbahnstrompfad eine separate Diode in Reihe zum Ansteuerelement geschaltet sein, vorzugsweise in Stromflussrichtung stromaufwärts.

Es ist außerdem vorteilhaft, wenn im ersten Auswertezweig parallel zum Einbahnstrompfad ein weiterer Parallelstrompfad vorhanden ist, indem beispielsweise ein Widerstand angeordnet sein kann. Wenn in der vorliegenden Anmeldung von einem Widerstand die Rede ist, ist ein Ohmscher Widerstand gemeint, solange nichts anderes angegeben ist. Der erste Schalter ist vorzugsweise in Reihe zum Einbahnstrompfad und zum Parallelstrompfad geschaltet.

Bei einer Ausführungsform der Erfindung ist in der Auswerteschaltung ein manuell betätigbarer zweiter Schalter vorhanden. Die beiden Schalter können so ausgeführt sein, dass sie unabhängig voneinander - also jeweils einzeln oder auch beide gleichzeitig - betätigbar sind. Alternativ dazu können die beiden Schalter auch mechanisch gekoppelt sein, so dass immer nur einer der beiden Schalter betätigt werden kann, wie es etwa bei einer Schaltwippe der Fall ist. Bei einem bevorzugten Ausführungsbeispiel sind der erste Schalter und der zweite Schalter jeweils als Taster ausgebildet, die in ihrem Ruhezustand in einem sperrenden Zustand sind und manuell in den leitenden Zustand umgeschaltet werden können.

Es ist außerdem vorteilhaft, wenn der zweite Schalter in einem zweiten Auswertezweig der Auswerteschaltung angeordnet ist. Der zweite Auswertezweig ist mit dem Auswerteanschluss verbunden. Vorzugsweise ist in Reihe zum zweiten Schalter ein Widerstand geschaltet.

Bei einem Ausführungsbeispiel sind der zweite Schalter und das Ansteuerelement über einen Verbindungsstrompfad gekoppelt. Beispielsweise kann der Verbindungsstrompfad eine permanente, nicht sperrbare elektrische Verbindung zwischen dem zweiten Schalter und dem Ansteuerelement herstellen. Der Verbindungsstrompfad kann bauteilfrei ausgebildet sein. Insbesondere sind der zweite Schalter und der Verbindungsstrompfad parallel zum Ansteuerelement und dem ersten Schalter geschaltet. Bei geschlossenem bzw. leitendem zweiten Schalter wird eine niederohmige Überbrückung des Ansteuerelements bewirkt. Bei leitendem zweiten Schalter kann somit kein Stromfluss durch das Ansteuerelement erfolgen. Unter einer niederohmigen Überbrückung des Ansteuerelements ist eine Überbrückung zu verstehen, die einen derart geringen Widerstandswert hat, dass der Spannungsabfall an der Überbrückung im Wesentlichen gleich null ist und insbesondere kleiner ist als eine Aktivierungsspannung des Ansteuerelements, beispielsweise die Flussspannung einer Leuchtdiode.

Es ist außerdem vorteilhaft, wenn der erste Koagulationsausgang der Betriebsschaltung schalterlos mit dem Versorgungseingang verbunden ist. In dieser Verbindung kann beispielsweise ein Kondensator angeordnet sein.

Vorzugsweise hat die Betriebsschaltung eine Transformatorschaltung mit einem Transformator. Die Transformatorschaltung ist primärseitig mit dem Versorgungseingang und sekundärseitig mit dem Schneidausgang verbunden, wobei diese Verbindungen mittelbar oder unmittelbar sein können. Der Transformator der Transformatorschaltung kann ohne galvanische Trennung als Spartransformator ausgeführt sein. Der Transformator kann alternativ auch eine von der Sekundärseite galvanisch getrennte Primärseite aufweisen. Die Transformatorschaltung ist insbesondere dazu eingerichtet, die am Versorgungsanschluss anliegende Versorgungsspannung zu erhöhen, beispielsweise von etwa 100 Volt auf etwa 450 Volt Wechselspannung.

Ein Verfahren, das nicht zum beanspruchten Teil der Erfindung gehört, zum Betreiben eines Instruments zur Koagulation und Dissektion von biologischem Gewebe, insbesondere unter Verwendung des vorstehend beschriebenen Instruments, weist folgende Schritte auf:

Zunächst wird ein Auswertesignal mit ersten Halbwellen einer Polarität und zweiten Halbwellen der jeweils anderen Polarität an den Auswerteanschluss angelegt. Anschließend wird ausgewertet, dass der erste Schalter in seinem leitenden Zustand oder seinem sperrenden Zustand ist. Diese Auswertung findet während wenigstens einer der ersten Halbwellen statt. Während wenigstens einer der nachfolgenden zweiten Halbwellen wird abhängig vom Auswerteergebnis ein Ansteuersignal für die Umschalteinheit erzeugt. Insbesondere wird dann, wenn festgestellt wurde, dass der erste Schalter manuell betätigt wurde, eine Versorgungsspannung an den Versorgungseingang angelegt. Das Instrument erzeugt ein Ansteuersignal, das die Umschalteinheit bei betätigtem ersten Schalter in den ersten Schaltzustand umschaltet, in dem der Schneidausgang - vorzugsweise über eine Transformatorschaltung - mit dem Versorgungsanschluss verbunden ist, so dass eine elektrische Spannung oder ein elektrischer Strom an dem Schneidausgang bereitgestellt wird, der für die Dissektion geeignet ist.

Insbesondere wird eine für die Dissektion geeignete Spannung oder ein für die Dissektion geeigneter Strom nicht an die Schneidelektrode angelegt, wenn erkannt wurde, dass der zweite Schalter des Instruments betätigt wurde, unabhängig vom Betätigungszustand des ersten Schalters. In diesem Fall wird ein Ansteuersignal erzeugt, dass die Umschalteinheit in den zweiten Schaltzustand umschaltet.

Vorteilhafte Ausgestaltungen der Erfindung ergeben sich aus den abhängigen Patentansprüchen, der Beschreibung und der Zeichnungen. Nachfolgend werden bevorzugte Ausführungsbeispiele der Erfindung anhand der beigefügten Zeichnungen im Einzelnen erläutert. Es zeigen:
Figur 1 eine schematische Darstellung einer Vorrichtung 20 zur Koagulation und Dissektion mit einem Gerät und einem elektrisch mit dem Gerät verbundenen Instrument,
Figur 2 eine perspektivische Teildarstellung eines Werkzeugs des Instruments aus Figur 1,
Figur 3 einen Schaltplan eines Ausführungsbeispiels einer Betriebsschaltung des Instruments,
Figur 4 eine schematische Darstellung eines zeitlichen Verlaufs eines beispielhaften Auswertesignals,
Figuren 5-12 einen Schaltplan eines Ausführungsbeispiels einer Auswerteschaltung der Betriebsschaltung aus Figur 3 in jeweils unterschiedlichen Zuständen,
Figur 13 einen Schaltplan einer optionalen Spannungserhöhungsschaltung für die Betriebsschaltung aus Figur 3,
Figur 14 ein abgewandeltes Ausführungsbeispiel zur Realisierung einer Diodenfunktion mittels eines Transistors und
Figur 15 ein Schaltplan für ein abgewandeltes Ausführungsbeispiel für eine Umschalteinheit für die Betriebsschaltung aus Figur 3.

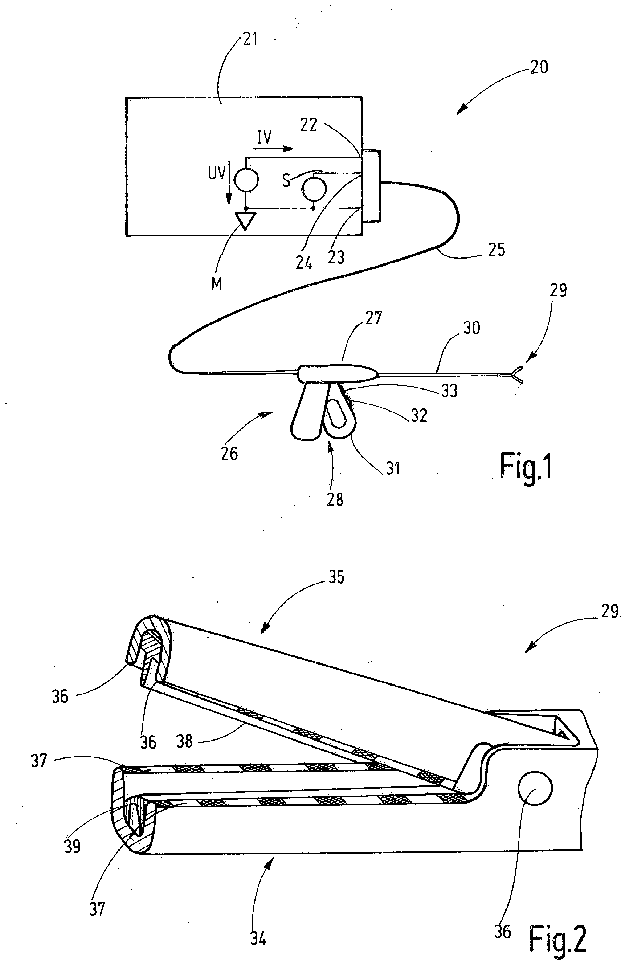

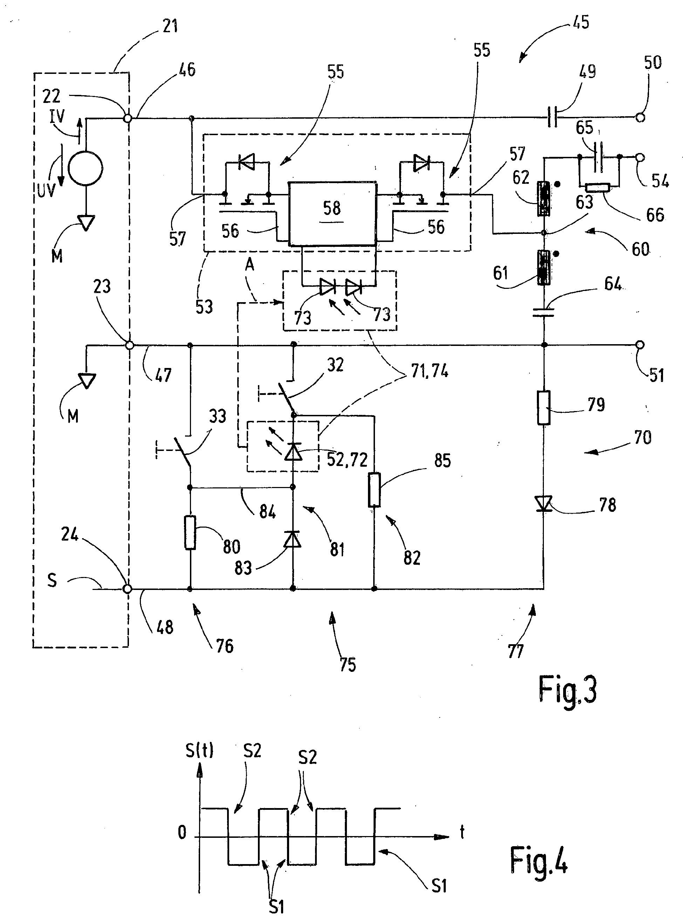

In Figur 1 ist eine Vorrichtung 20 zur Koagulation und Dissektion veranschaulicht. Die Vorrichtung 20 hat ein Gerät 21, das an einem ersten Geräteausgang 22 eine Versorgungsspannung UV oder einen Versorgungsstrom IV bereitstellt. Ein zweiter Geräteausgang 23 ist mit Masse M verbunden. An einem dritten Geräteausgang 24 wird ein Auswertesignal S bereitgestellt. Das Auswertesignal S ist beim Ausführungsbeispiel ein periodisches Signal, beispielsweise eine Spannung oder ein Strom, mit ersten Halbwellen S1 einer Polarität und zweiten Halbwellen S2 einer jeweils anderen Polarität. Beim Ausführungsbeispiel sind die ersten Halbwellen S1 negativ und die zweiten Halbwellen S2 positiv, wie es schematisch in Figur 4 veranschaulicht ist. Das Auswertesignal S ist in Figur 4 als Rechtecksignal veranschaulicht. Alternativ dazu könnte das Auswertesignal S auch irgendwelche anderen Wellenformen aufweisen, beispielsweise eine sinusförmige oder eine dreieckförmige Wellenform mit positiven und negativen Halbwellen.

Das Auswertesignal S ist vorzugsweise periodisch. In Abwandlung zum veranschaulichten Ausführungsbeispiel kann das Auswertesignal S aber auch aperiodisch sein. Es ist außerdem möglich, dass die Zeitdauer für eine erste Halbwelle und die Zeitdauer für eine zweite Halbwelle unterschiedlich lang sind. In Figur 4 haben sowohl die ersten Halbwellen S1, als auch die zweiten Halbwellen S2 dieselbe zeitliche Länge, so dass jede Halbwelle S1, S2 einer halben Periodendauer entspricht.

An das Gerät 21 ist über ein mehradriges Kabel 25 ein Instrument 26 zur Koagulation und Dissektion angeschlossen. Das Instrument 26 hat ein Gehäuse 27 mit Handgriff 28 sowie ein Werkzeug 29. Das Werkzeug 29 ist beim Ausführungsbeispiel über ein Verbindungsteil 30 mit dem Gehäuse 27 verbunden. Das Verbindungsteil 30 kann stabförmig ausgeführt sein. An dem Handgriff 28 ist ein Bedienelement 31 für das Werkzeug 29 vorhanden. Das Bedienelement 31 dient zur mechanischen und elektrischen Betätigung des Werkzeugs 29. An dem Bedienteil 28 ist ein manuell betätigbarer erster Schalter 32 sowie ein manuell betätigbarer zweiter Schalter 33 vorhanden. Die beiden Schalter 32, 33 sind beim Ausführungsbeispiel als Taster ausgebildet und befinden sich in ihrem nicht betätigten Ruhezustand in einem elektrisch sperrenden Zustand.

In Figur 2 ist das Werkzeug 29 des Instruments 26 aus Figur 1 in einer perspektivischen Teildarstellung veranschaulicht. Das Werkzeug 29 hat zwei relativ zueinander bewegbare Branchen 34, 35, die in einem Gelenk 36 gelenkig miteinander verbunden sind. Über das Bedienelement 31 können die Branchen relativ zueinander geschwenkt werden. An der einen Branche ist wenigstens eine erste Koagulationselektrode 36 angeordnet, während an die jeweils anderen Branchen wenigstens eine zweite Koagulationselektrode 37 vorhanden ist. Jeweils eine erste Koagulationselektrode 36 und eine zugeordnete zweite Koagulationselektrode 37 bilden ein Koagulationselektrodenpaar. An den Branchen 34, 35 können mehrere Koagulationselektrodenpaare vorhanden sein.

Außerdem verfügt eine der Branchen zusätzlich über eine Schneidelektrode 38. Die Schneidelektrode 38 ist beim Ausführungsbeispiel als Einsatz in einer nutförmigen Vertiefung an der Branche 35 angeordnet und durch zwei erste Koagulationselektroden 36 flankiert. Die Schneidelektrode 38 ist von den ersten Koagulationselektroden 36 elektrisch isoliert am Werkzeug 39 angeordnet. Die mehreren ersten Koagulationselektroden 36 können elektrisch miteinander verbunden sein.

Der Branche 34 ist ein Widerlager 39 für die Schneidelektrode 38 angeordnet. Wird das Werkzeug 29 über das Bedienelement 31 geschlossen, liegen sich jeweils eine erste Koagulationselektrode 36 und eine zweite Koagulationselektrode 37 gegenüber. Die Schneidelektrode 38 liegt benachbart oder am Widerlager 39 an. Über die Schalter 32, 33 können die elektrischen Funktionen der Elektroden 36, 37, 38 bedient werden.

Ein Ausführungsbeispiel einer Betriebsschaltung 45 des Instruments 26 ist in Figur 3 veranschaulicht. Die Betriebsschaltung 45 hat einen Versorgungsanschluss 46, mittels dem die Betriebsschaltung 45 an den ersten Geräteausgang 22 anschließbar ist sowie einen Masseanschluss 47, mittels dem die Betriebsschaltung 45 mit dem dritten Geräteausgang 24 verbindbar ist. Ein Auswerteanschluss 48 der Betriebsschaltung 45 ist mit dem dritten Geräteausgang 24 verbindbar. Am Auswerteanschluss 48 liegt daher das Auswertesignal S des Geräts 21 an. Über den Masseanschluss 47 wird die Betriebsschaltung 45 mit Masse M verbunden. Der Versorgungsanschluss 46 wird an eine HF-Stromquelle oder HF-Spannungsquelle des Geräts 21 angelegt, um einen Versorgungsstrom IV oder eine Versorgungsspannung UV für die Betriebsschaltung 45 bereitzustellen.

Der Versorgungsanschluss 46 ist über einen ersten Kondensator 49 mit einem Koagulationsausgang 50 verbunden. Der erste Koagulationsausgang 50 ist mit der wenigstens einen ersten Koagulationselektrode 46 verbunden. Ein zweiter Koagulationsausgang 51 ist mit der wenigstens einen zweiten Koagulationselektrode 47 verbunden. Der zweite Koagulationsausgang 51 ist beim Ausführungsbeispiel als Masseausgang ausgeführt. Der zweite Koagulationsausgang 51 ist dazu mit dem Masseanschluss 47 uns mithin mit der Masse M verbunden.

Die Betriebsschaltung 45 weist außerdem eine mittels eines Ansteuersignals A eines Ansteuerelements 52 umschaltbare Umschalteinheit 53 auf. Die Umschalteinheit 53 ist in die elektrische Verbindung zwischen dem Versorgungsanschluss 46 und einem Schneidausgang 54 geschaltet. Die Umschalteinheit 53 ist dazu eingerichtet, am Schneidausgang 54 in einem ersten Schaltzustand eine Spannung und/oder einen Strom bereitzustellen, der für eine Dissektion unter Verwendung der Schneidelektrode 38 eingerichtet ist und in einem zweiten Schaltzustand an dem Schneidausgang 54 im Wesentlichen keine elektrische Energie bereitzustellen. Im zweiten Schaltzustand wird beispielsgemäß die Verbindung zwischen dem Versorgungsanschluss 46 und dem Schneidausgang 54 unterbrochen.

Die Umschalteinheit 53 ist ohne mechanische Schalter ausgeführt und weist wenigstens einen Halbleitschalter 55 und beim Ausführungsbeispiel zwei in Reihe geschaltete Halbleiterschalter 55 auf. Beispielsgemäß ist jeder Halbleiterschalter 55 durch einen Feldeffekttransistor gebildet, der hier als normal sperrender n-Kanal MOSFET veranschaulicht ist. Jeder Halbleiterschalter 55 hat einen Steueranschluss 56, der beim Ausführungsbeispiel durch das Gate der MOSFETS gebildet ist.

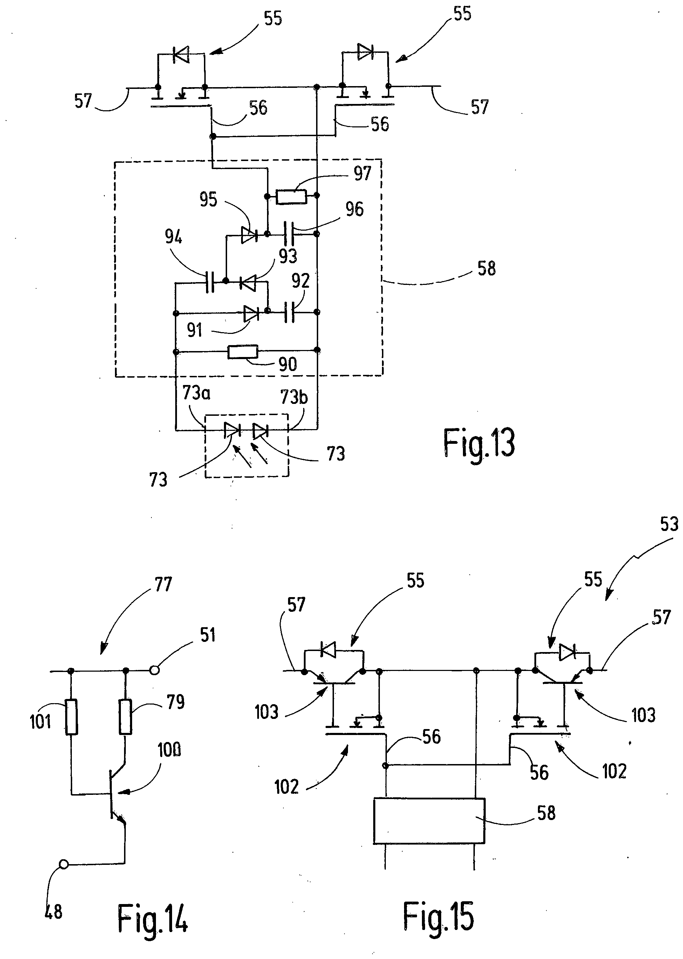

Die beiden Steueranschlüsse 56 sind vorzugsweise miteinander verbunden. Außerdem können die beiden Source-Anschlüsse der MOSFETS miteinander verbunden sein (Fig. 13) .

Die Drain-Anschlüsse der beiden MOSFETS bilden jeweils einen Schaltanschluss 57 der Umschalteinheit 53. Zwischen den beiden Schaltanschlüssen 57 ist die Schaltstrecke der Umschalteinheit 53 gebildet. Der eine Schaltanschluss 57 der Umschalteinheit 53 ist mit dem Versorgungsanschluss 46 verbunden. Der jeweils andere Schaltanschluss 57 ist mit dem Schaltausgang 54 verbunden.

Die Umschalteinheit 53 weist beispielsgemäß eine Lade- und Entladeschaltung 58 für den wenigstens einen Halbleitschalter 55 auf. Die Lade- und Entladeschaltung 58 ist dazu eingerichtet elektrische Ladungen an den Steueranschlüssen 56 ausreichend lange halten zu können, um den ersten Schaltzustand der Umschalteinheit 53 von einer ersten Halbwelle S1 mindestens bis zur nächsten ersten Halbwelle S1 aufrechterhalten zu können, solange der erste Schalter 32 betätigt ist. Andererseits ist die Lade- und Entladeschaltung 58 dazu eingerichtet, die an Steueranschlüssen 56 vorhandenen Ladungen abzuführen, so dass die Halbleiterschalter 55 auch wieder in ihren nicht leitenden Zustand übergehen können, wenn der erste Schalter 32 nicht mehr betätigt wird.

Die Betriebsschaltung 45 hat beim Ausführungsbeispiel eine Transformatorschaltung mit einem Transformator 60. Der Transformator 60 weist eine Primärwicklung 61 und eine Sekundärwicklung 62 auf. Beim Ausführungsbeispiel ist der Transformator 60 als Spartransformator ausgebildet. Die Primärwicklung 61 und die Sekundärwicklung 62 sind dabei in Reihe geschaltet und ein Mittelabgriff 63 ist mit einem zugeordneten Schaltanschluss 57 der Umschalteinrichtung 53 verbunden. Ausgehend vom Mittelabgriff 63 ist eine Reihenschaltung aus der Primärwicklung 61 und einem zweiten Kondensator 64 mit Masse M verbunden. Ausgehend vom Mittelabgriff 63 ist eine Reihenschaltung aus der Sekundärwicklung 62 und einem dritten Kondensator 65 mit dem Schneidausgang 54 verbunden. Parallel zum dritten Kondensator 65 ist ein erster Widerstand 66 geschaltet. Die Parallelschaltung aus dem dritten Kondensator 65 und dem ersten Widerstand 66 bildet eine Funkenerkennungsschaltung. Treten Funken auf, hat der Versorgungsstrom IV bzw. die Versorgungsspannung UV einen Gleichanteil, was in dem Gerät 21 ausgewertet und erkannt werden kann. Ist eine solche Funkenerkennung nach Funkenerkennung nicht gewünscht, kann die Funkenerkennungsschaltung auch entfallen.

Die Betriebsschaltung 45 verfügt außerdem über eine Auswerteschaltung 70, zu der das Ansteuerelement 52 gehört. Die Auswerteschaltung 70 ist über eine Kopplungseinrichtung 71 mit der Umschalteinheit 53 gekoppelt, um das Ansteuersignal A von der Auswerteschaltung 70 zu der Umschalteinheit 53 zu übertragen. Die Kopplungseinrichtung 71 hat hierfür wenigstens ein Senderbauteil 72 in der Auswerteschaltung 70 und wenigstens ein Empfängerbauteil 73, das mit dem wenigstens einen Steueranschluss 56 der Umschalteinheit 53 verbunden ist. Beim Ausführungsbeispiel ist das wenigstens eine Senderbauteil 72 eine Leuchtdiode und das wenigstens eine Empfängerbauteil 73 eine Fotodiode oder alternativ ein Fototransistor. Die Kopplungseinrichtung 71 kann somit durch einen Optokoppler 74 gebildet sein. Bei dem hier gezeigten Ausführungsbeispiel ist das Senderbauteil 72 durch das Ansteuerelement 52 gebildet.

Die Auswerteschaltung 70 weist bei dem in Figur 3 gezeigten Ausführungsbeispiel einen ersten Auswertezweig 75 mit dem ersten Schalter 32, einen zweiten Auswertezweig 76 mit dem zweiten Schalter 33 sowie einen dritten Auswertezweig 77 auf. Die drei Auswertezweige 75, 76, 77 sind parallel zueinander zwischen den Masseanschluss 47 und den Auswerteanschluss 48 geschaltet.

Der dritte Auswertezweig 77 ist beim Ausführungsbeispiel durch eine Reihenschaltung aus einer ersten Diode 78 und einem zweiten Widerstand 79 gebildet. Die Kathode der ersten Diode 78 ist mit dem Auswerteanschluss 58 und die Anode mit dem zweiten Widerstand 79 verbunden. Der zweite Widerstand 79 ist mit seinem anderen Anschluss mit Masse M verbunden.

Der zweite Auswertezweig 76 weist zusätzlich zum zweiten Schalter 33 einen dritten Widerstand 80 auf, der in Reihe zum zweiten Schalter 33 geschaltet ist.

Der erste Auswertepfad 75 hat in Reihe zum ersten Schalter 72 einen Einbahnstrompfad 81 sowie einen parallel zum Einbahnstrompfad geschalteten Parallelstrompfad 82. Im Einbahnstrompfad 81 ist das Ansteuerelement 52 in Reihe zum ersten Schalter 32 geschaltet. Der Einbahnstrompfad 81 weist mit wenigstens einem Bauteil mit Diodenfunktion auf, so dass ein Strom durch den Einbahnstrompfad 81 nur in eine Richtung fließen kann und beispielsgemäß vom Auswerteanschluss 48 zum Masseanschluss 47, sofern der erste Schalter 32 geschlossen ist. Bei dem hier dargestellten Ausführungsbeispiel ist in Reihe zu dem Ansteuerelement 52 ein zusätzliches Bauteil mit Diodenfunktion geschaltet, beispielsgemäß eine zweite Diode 83. Die Anode der zweiten Diode 83 ist mit dem Auswerteanschluss 48 verbunden und die Kathode der zweiten Diode 83 ist mit dem Ansteuerelement 52 verbunden und beispielsgemäß mit der Anode der das Ansteuerelement 52 bildenden Leuchtdiode.

Außerdem ist der Verbindungspunkt zwischen der zweiten Diode 83 und dem Ansteuerelement 52 bei dem veranschaulichten Ausführungsbeispiel über einen Verbindungsstrompfad 84 mit dem zweiten Schalter 33 verbunden, so dass der Verbindungsstrompfad 84 und der zweite Schalter 33 parallel zum Ansteuerelement 52 und dem ersten Schalter 32 geschaltet sind.

Im Parallelstrompfad 82 ist parallel zur zweiten Diode 83 und dem Ansteuerelement 72 ein vierter Widerstand 85 geschaltet.

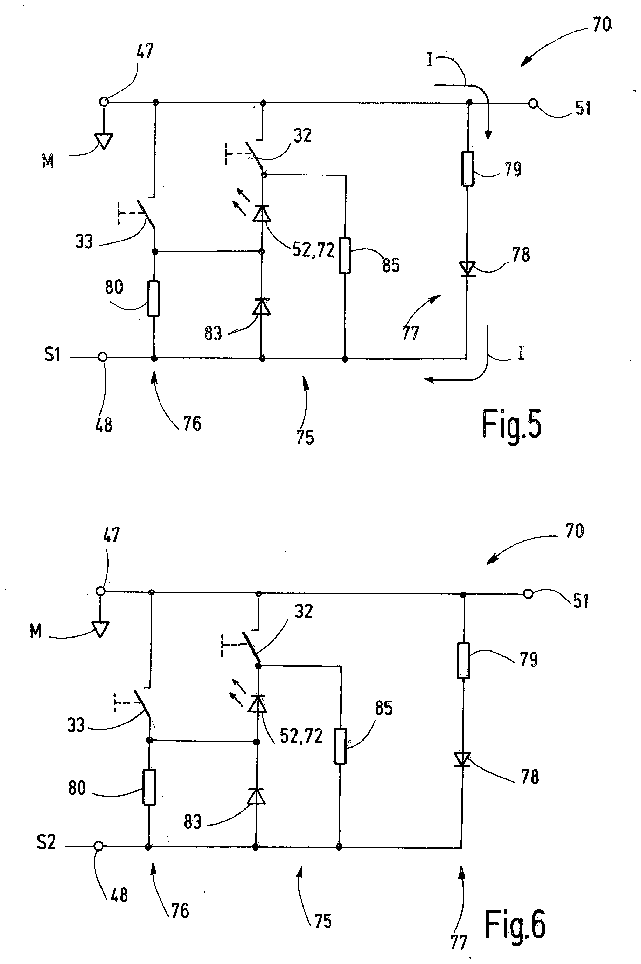

Die Funktionsweise der Betriebsschaltung 45 wird nachfolgend anhand der Figuren 5 bis 12 erläutert. Zur Unterscheidung wird dabei ein durch den ersten Auswertezweig 75 fließender Strom als erster Strom I1, ein durch den zweiten Auswertezweig 76 fließender Strom als zweiter Strom I2 und ein durch den dritten Auswertezweig 77 fließender Strom als dritter Strom I3 bezeichnet. Die Widerstandswerte der Widerstände 79, 85 und 80 in den Auswertezweigen 75, 76, 77 werden so gewählt, dass das Gerät 21 am Betrag der Ströme I1, I2, I3 erkennt, ob und wenn ja welcher Schalter 32, 33 in seinem leitenden Zustand ist. Insbesondere sind die Widerstandswerte des dritten Widerstands 80 und des vierten Widerstands 85 unterschiedlich groß. Dadurch ist im Gerät 21 eine Erkennung möglich, welcher der Schalter 32, 33 während einer ersten Halbwelle S1 leitet bzw. welcher der Schalter 32, 33 sperrt.

Es sei angenommen, dass beide Schalter 32, 33 der Auswerteschaltung 70 (z.B. in ihrem jeweiligen Ausgangszustand) nicht leitend sind, wie es in den Figuren 5 und 6 veranschaulicht ist. Während den ersten Halbwellen S1 des Auswertesignales S fliest ein dritter Strom I3 lediglich durch den dritten Auswertepfad 77 und mithin durch den zweiten Widerstand 79 und die erste Diode 78 (Figur 5). Das Gerät 21 kann durch Auswertung des dritten Stromes I3 erkennen, dass die anderen beiden Auswertepfade 75, 76 unterbrochen sind und somit weder koaguliert noch geschnitten werden soll. Während der zweiten Halbwellen S2 fliest kein Strom durch die Auswerteschaltung 70 (Figur 6).

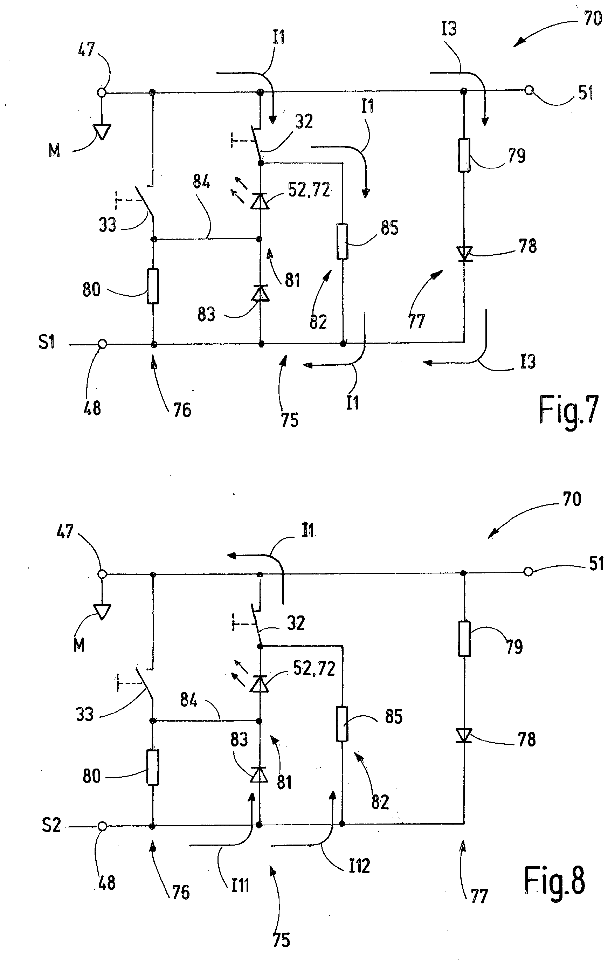

Es sei jetzt angenommen, dass ein Bediener des Instruments den ersten Schalter 32 betätigt, so dass dieser in seinen leitenden Zustand umgeschaltet wird, was in den Figuren 7 und 8 veranschaulicht ist. Während der ersten Halbwellen S1 fliest ein erster Strom I1 durch den ersten Auswertezweig 75 und ein dritter Strom I3 durch den dritten Auswertezweig 77 (Figur 7). Der erste Strom I1 fliest dabei durch den ersten Schalter 33 und den Parallelstrompfad 82, also durch den vierten Widerstand 85. Während einer oder mehrerer erster Halbwellen S1 kann das Gerät 21 daher erkennen, dass der erste Schalter 32 betätigt wurde und sich in seinem leitenden Zustand befindet, während der zweite Schalter 33 nicht leitend ist.

Nachdem erkannt wurde, dass der erste Schalter 32 betätigt wurde, wird am ersten Geräteausgang 22 und mithin am Versorgungsanschluss 46 die Versorgungsspannung UV bzw. der Versorgungsstrom IV bereitgestellt.

Während einer darauffolgenden zweiten Halbwelle S2 (positiver Halbwelle), kann aufgrund der ersten Diode 78 kein Strom durch den dritten Auswertezweig 77 fliesen. Der erste Strom I1 durch den ersten Auswertezweig 75 weist einen Teilstrom I11 durch den Einbahnstrompfad 81 und einen Teilstrom I12 durch den Parallelstrompfad 82 auf. Der Teilstrom I11 durch den Einbahnstrompfad 81 ist wegen des geringeren Widerstandswertes gegenüber dem vierten Widerstand 85 deutlich größer als der Teilstrom I12 durch den Parallelstrompfad 82. Der Teilstrom I11 fliest durch das Ansteuerelement 52, das gleichzeitig das Senderbauteil 72 darstellt. Das Ansteuersignal A wird an das wenigstens eine Empfängerbauteil 73 übermittelt und schaltet die Umschalteinheit 53 in den ersten Schaltzustand um, in dem der wenigstens eine Halbleitschalter 55 leitet und eine elektrische Verbindung zwischen den Schaltanschlüssen 57 herstellt. Das Ansteuersignal A wird beispielsgemäß durch das von der Leuchtdiode (Senderbauteil 72) des Optokopplers 74 übertragene Licht gebildet, das dafür sorgt, dass die beispielsgemäß zwei Fotodioden des Optokopplers 74 eine Spannung erzeugen, die als Quelle für das Erzeugen einer Drain-Source-Spannung oder Basis-Emitter-Spannung durch die Lade- und Entladeschaltung 58 dient und den wenigstens einen Halbleitschalter 55 dadurch in den leitenden Zustand umschaltet. Der Versorgungsanschluss 46 ist daher über den Transformator 60 mit dem Schneidausgang 54 verbunden und am Schneidausgang 54 wird eine durch den Transformator 60 erhöhte Spannung zur Dissektion bereitgestellt.

Das wenigstens eine Empfängerbauteil 73 ist mit dem Steueranschluss 56 jedes Halbleitschalters 55 über die Lade- und Entladeschaltung 58 verbunden, um einerseits die Ladungen in den Steueranschlüssen 56 ausreichend lange halten zu können (zumindest während der Zeitdauer einer ersten Halbwelle S1) und andererseits die dort vorhandenen Ladungen auch wieder abführen zu können, wenn die Umschalteinheit 53 in den zweiten Schaltzustand umgeschaltet werden soll.

Über die Lade- und Entladeschaltung 58 wird die Ladung beim Ausführungsbeispiel in den Gate-Anschlüssen der MOSFETs aufrecht erhalten, so dass die Umschalteinheit 53 in ihrem zweiten Schaltzustand (leitender Zustand) bleibt, auch wenn eine erste Halbwelle S1 anliegt. Zumindest für die Dauer einer ersten Halbwelle S1 wird die Ladung in den Gates der MOSFETs durch die Lade- und Entladeschaltung 58 aufrecht erhalten, wenn die Fotodioden des Optokopplers 74 bei leitendem ersten Schalter 32 während einer zweiten Halbwelle S2 erneut durch die Leuchtdiode des Optokopplers 74 angesteuert werden.

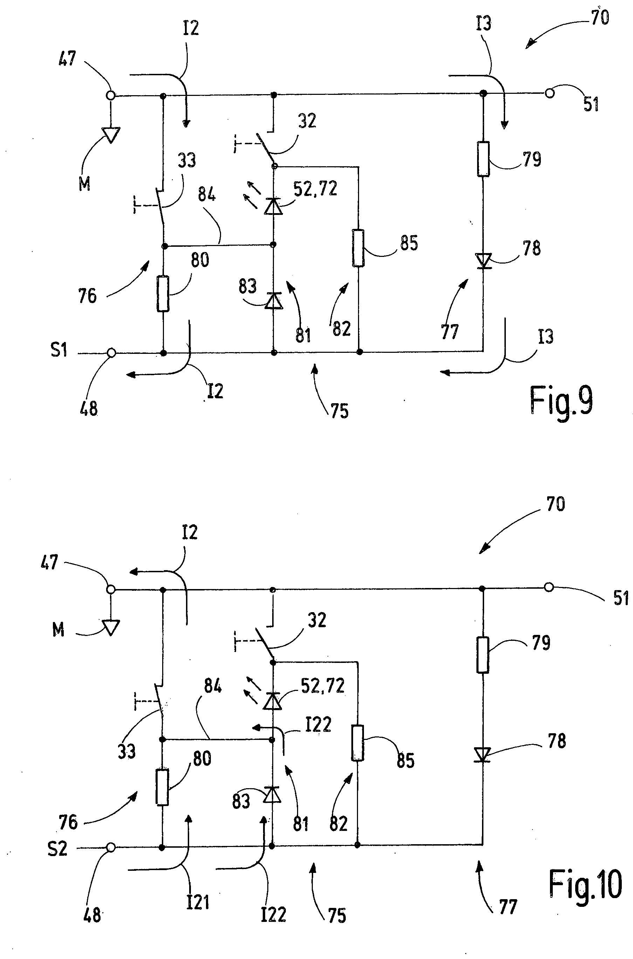

In den Figuren 9 und 10 ist ein weiterer Zustand dargestellt, bei dem der erste Schalter 32 nicht leitet, jedoch der zweite Schalter 33 betätigt wurde und sich daher in seinem leitenden Zustand befindet. Während der ersten Halbwellen S1 des Auswertesignals S fliest ein zweiter Strom I2 durch den zweiten Auswertezweig 76 sowie ein dritter Storm I3 durch den dritten Auswertezweig 77 (Figur 9). Das Gerät 21 erkennt, dass dabei die Ströme durch den zweiten Widerstand 79 und den dritten Widerstand 80 fliesen und kann dadurch feststellen, dass der zweite Schalter 33 leitend, der erste Schalter 32 jedoch nicht leitend und damit unbetätigt ist. Das Gerät 21 stellt daraufhin am ersten Geräteausgang 22 und mithin am Versorgungsanschluss 46 die Versorgungsspannung UV bzw. den Versorgungsspannung IV bereit, die somit am ersten Koagulationsausgang 50 bereitsteht.

Die Umschalteinheit 53 bleibt in ihrem zweiten Schaltzustand, in dem an dem Schneidausgang 54 keine Spannung bzw. kein Strom bereitgestellt wird. Dies wird dadurch erreicht, dass während der zweiten Halbwellen S2 ein zweiter Strom I2 durch den zweiten Schalter 33 fliest, wobei ein Teilstrom I21 des zweiten Stroms I2 durch den dritten Widerstand 80 und ein anderer Teilstrom I22 des zweiten Stroms I2 durch die zweite Diode 83 und den Verbindungsstrompfad 84 fliest. Jedenfalls ist das Ansteuerelement 52 bzw. das Senderbauteil 72 stromlos, so dass die Halbleiterschalter 55 in ihrem nicht leitenden Zustand bleiben (Figur 10) .

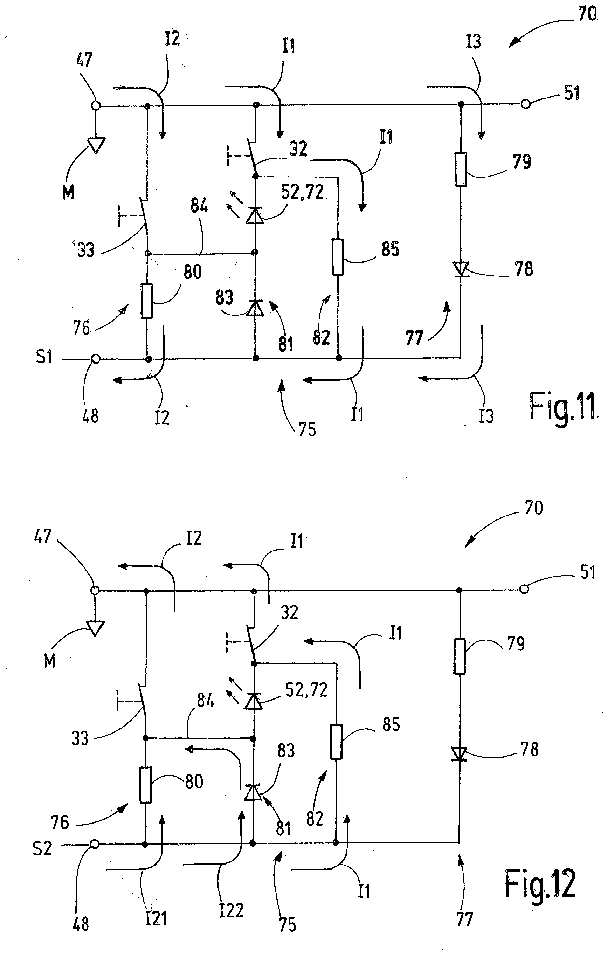

Es sei nunmehr angenommen, dass ein Bediener sowohl den ersten Schalter 32, als auch den zweiten Schalter 33 betätigt und daher beide Schalter 32, 33 ihren leitenden Zustand annehmen (Figuren 11 und 12). Während der ersten Halbwellen S1 (Figur 11) fliest ein erster Strom I1 durch den ersten Auswertezweig 75, wobei der erste Strom I1 durch den ersten Schalter 32 und den Parallelstromzweig 82 fliest. Ein zweiter Strom I2 fliest durch den zweiten Auswertezweig 76 und ein dritter Strom I3 fliest durch den dritten Auswertezweig 77. Es wird somit erkannt, dass wenigstens einer der Schalter 32, 33 betätigt ist und das Gerät 21 stellt am ersten Geräteausgang 22 eine Versorgungsspannung UV bzw. einen Versorgungsstrom IV bereit.

Während einer zweiten Halbwelle S2 (Figur 12) fliest ein zweiter Strom I2 durch den ersten Schalter 33 des zweiten Auswertezweiges 76, der sich aus einem Teilstrom I21 durch den dritten Widerstand 80 und einem Teilstrom I22 durch die zweite Diode 83 und den Verbindungsstrompfad 84 zusammensetzt. Ein erster Strom I1 fliest durch den Parallelstrompfad 82 und den ersten Schalter 32. Das Ansteuerelement 52 bzw. das Sendeelement 72 ist stromlos und somit bleibt die Umschalteinheit 53 in ihrem ersten Schaltzustand und am Schneidausgang 54 wird keine Spannung bzw. kein Strom für das Schneiden bereitgestellt. Das Ansteuerelement 52 bzw. das Sendeelement 72 wird durch den zweiten Schalter 33 niederohmig überbrückt bzw. kurzgeschlossen. Somit wird ohne mechanische Mittel durch den Aufbau der Auswerteschaltung 70 vermieden, dass bei der Betätigung des zweiten Schalters 33 eine Schneidspannung an der Schneidelektrode 38 anliegt. Ein versehentliches Schneiden ist daher nicht möglich, wenn der zweite Schalter 33 zur Anforderung des Koagulierens betätigt wird.

In Figur 13 ist ein Ausführungsbeispiel einer Lade- und Entladeschaltung 58 veranschaulicht. Es versteht sich, dass auch andere Lade- und Entladeschaltungen verwendet werden können.

Das wenigstens einen Empfängerbauteil 73 hat einen ersten Anschluss 73a höheren elektrischen Potenzials (hier: Anodenseite der wenigstens einen Fotodiode) und einen zweiten Anschluss 73b niedrigeren elektrischen Potenzials (hier: Kathodenseite der wenigstens einen Fotodiode). Am ersten Anschluss 73a liegt bei einer Ansteuerung durch das Senderbauteil 72 ein höheres Potenzial an als am zweiten Anschluss 73b.

Parallel zum wenigstens einen Empfängerbauteil 73 ist ein fünfter Widerstand 90 geschaltet. An den ersten Anschluss 73a ist eine Anode einer dritten Diode 91 angeschlossen, deren Kathode mit einem vierten Kondensator 92 verbunden ist. Die andere Seite des vierten Kondensators 92 ist mit dem zweiten Anschluss 73b verbunden. Der fünfte Widerstand 90 ist parallel zu der Reihenschaltung aus der dritten Diode 91 und dem vierten Kondensator 92 geschaltet.

Eine Reihenschaltung aus einer vierten Diode 93 und einem fünften Kondensator 94 ist parallel zur dritten Diode 91 geschaltet, wobei die Anode der vierten Diode 93 mit der Kathode der dritten Diode 91 verbunden ist. Die Kathode der vierten Diode 93 ist mit einer Anode einer fünften Diode 95 verbunden. Die Kathode der fünften Diode 95 ist mit einem sechsten Kondensator 96 verbunden. Der andere Anschluss des sechsten Kondensators 96 ist mit dem zweiten Anschluss 73b verbunden. Außerdem ist der sechste Kondensator 96 zwischen die Steueranschlüsse 56 (Drain-Anschlüsse) und den Verbindungspunkt zwischen den beiden in Reihe geschalteten Halbleiterschaltern 55 (Source-Anschlüsse) geschaltet. Parallel zum sechsten Kondensator 96 ist ein sechster Widerstand 97 geschaltet.

Die mehreren Kaskaden aus jeweils einer Diode 91, 93, 95 und einem in Reihe geschalteten Kondensator 92, 94, 96 dienen zur Spannungsvervielfachung der an dem wenigstens einen Empfängerbauteil 73 bei der Ansteuerung durch das Senderbauteil 72 anliegenden Spannung. Durch die vorhandenen Kondensatoren kann die Ansteuerung der Halbleitschalter 55 und beispielsgemäß die Ladung in den Gates der Feldeffekttransistoren aufrechterhalten werden, auch wenn während einer ersten Halbwelle S1 kurzzeitig keine Spannung an dem wenigstens einen Empfängerbauteil 73 anliegt. Die Kondensatoren dienen somit als Pufferkondensatoren. Um ein Entladen zu ermöglichen, sind die Widerstände 90, 97 der Lade- und Entladeschaltung 58 vorhanden. Nachdem der erste Schalter 32 unbetätigt ist, können sich die Ladungen in den Ansteueranschlüssen 56 über die Widerstände 90, 97 ausgleichen und die Halbleitschalter 55 können in ihren sperrenden Zustand zurückkehren. Die Zeitdauer ab dem Umschalten des ersten Schalters 32 in den nicht leitenden Zustand bis zum Sperren der Halbleitschalter 55 hängt von der Dimensionierung der in der Lade- und Entladeschaltung 58 vorhandenen Bauteile ab.

In Abwandlung zu der vorstehend beschriebenen Lade- und Entladeschaltung 58 können auch mehr oder weniger Kaskaden von Dioden und Kondensatoren verwendet werden. Dies hängt davon ab, welche Spannung für das Durchsteuern der Halbleiterschalter 55 benötigt wird.

In Figur 14 ist eine abgewandelte Ausführungsform des dritten Auswertezweiges 77 veranschaulicht anstelle der ersten Diode 78 kann ein Transistor, insbesondere Bipolartransistor verwendet werden, dessen Kollektoranschluss mit dem zweiten Widerstand 79 verbunden ist und dessen Emitteranschluss mit dem Auswerteanschluss 48 verbunden ist. Der Basisanschluss des Transistors ist über einen siebten Widerstand 101 mit Masse verbunden.

In Figur 15 ist ein abgewandeltes Ausführungsbeispiel der Umschalteinheit 53 veranschaulicht. Die Halbleiterschalter 55 weisen Bipolartransistoren 103 auf und werden jeweils durch einen Halbleitersteuerschalter 102 angesteuert. Die Halbleitersteuerschalter 102 sind durch Feldeffekttransistoren beispielsgemäß durch normal sperrende n-Kanal MOSFETS gebildet. Die Steueranschlüsse 56 der Halbleitsteuerschalter 102 sind mit der Lade- und Entladeschaltung 58 verbunden. Außerdem sind die beiden Kollektoren der Bipolartransistoren 103 mit der Lade- und Entladeschaltung 58 verbunden. Jeder Emitter eines Bipolartransistors 103 bildet einen Schaltanschluss 57. Die Kollektoren der Bipolartransistoren 103 sind außerdem mit den Source-Anschlüssen, der die Halbleitersteuerschalter 102 bildenden Feldeffekttransistoren verbunden. Die Basis jedes Bipolartransistors 103 ist mit dem Drain-Anschluss eines jeweils zugeordneten Feldeffekttransistors verbunden. Sobald die Gate-Source-Spannung ausreichend groß ist, werden die Halbleitersteuerschalter 102 leitend, so dass ein Basisstrom aus den PNP-Bipolaransistoren 103 fliest und diese in ihren leitenden Zustand übergehen.

Die Erfindung betrifft ein Instrument zur wahlweisen Koagulation und Dissektion von biologischem Gewebe. Das Instrument hat ein Werkzeug 29 mit Koagulationselektroden 36 und wenigstens einer Schneidelektrode 38. Über eine Betriebsschaltung 45 werden die Elektroden 36, 38 angesteuert. Die Betriebsschaltung 45 hat eine Auswerteschaltung 70, der von einem externen Gerät 21 ein Auswertesignal S mit ersten Halbwellen S1 und zweiten Halbwellen S2 zugeführt wird. Die ersten Halbwellen S1 und die zweiten Halbwellen S2 haben entgegengesetzte Polaritäten. Während wenigstens einer ersten Halbwelle S1 wird durch die Auswerteschaltung 70 geprüft, ob an dem Instrument 26 ein erster Schalter 32 oder ein zweiter Schalter 33 oder beide Schalter 32, 33 betätigt sind. Abhängig vom Auswerteergebnis wird ein Ansteuersignal A erzeugt und an eine Umschalteinheit 53 übertragen, insbesondere unter Verwendung einer Kopplungseinrichtung 71 mit galvanischer Trennung. Abhängig vom Ansteuersignal A wird die Umschalteinheit 53 in einen ersten Schaltzustand oder einen zweiten Schaltzustand geschaltet. Im zweiten Schaltzustand wird keine zur Dissektion geeignete Spannung bzw. kein zur Dissektion geeigneter Strom an die Schneidelektrode 38 angelegt. Im ersten Schaltzustand wird eine für die Dissektion geeignete Spannung bzw. ein für die Dissektion geeigneter Strom an die Schneidelektrode 38 angelegt. Der erste Schaltzustand wird über das Ansteuersignal A vorzugsweise dann veranlasst, wenn die Auswerteschaltung 70 erkennt, dass nur der erste Schalter 32 betätigt ist.

### Bezugszeichenliste:

- 20: Vorrichtung zur Koagulation und Dissektion
- 21: Gerät
- 22: erster Geräteausgang
- 23: zweiter Geräteausgang
- 24: dritter Geräteausgang
- 25: Kabel
- 26: Instrument
- 27: Gehäuse
- 28: Handgriff
- 29: Werkzeug
- 30: Verbindungsteil
- 31: Bedienelement
- 32: erster Schalter
- 33: zweiter Schalter
- 34: Branche
- 35: Branche
- 36: erste Koagulationselektrode
- 37: zweiten Koagulationselektrode
- 38: Schneidelektrode

- 45: Betriebsschaltung
- 46: Versorgungsanschluss
- 47: Masseanschluss
- 48: Auswerteanschluss
- 49: erster Kondensator
- 50: erster Koagulationsausgang
- 51: zweiter Koagulationsausgang
- 52: Ansteuerelement
- 53: Umschalteinheit
- 54: Schneidausgang

- 55: Halbleiterschalter
- 56: Steueranschluss
- 57: Schaltanschluss
- 58: Lade- und Entladeschaltung

- 60: Transformator
- 61: Primärwicklung
- 62: Sekundärwicklung
- 63: Mittelabgriff
- 64: zweiter Kondensator
- 65: dritter Kondensator
- 66: erster Widerstand

- 70: Auswerteschaltung
- 71: Kopplungseinrichtung
- 72: Senderbauteil
- 73: Empfängerbauteil
- 73a: erster Anschluss
- 73b: zweiter Anschluss
- 74: Optokoppler
- 75: ersten Auswertezweig
- 76: zweiten Auswertezweig
- 77: dritten Auswertezweig
- 78: erste Diode
- 79: zweiten Widerstand
- 80: dritter Widerstand
- 81: Einbahnstrompfad
- 82: Parallelstrompfad
- 83: zweite Diode
- 84: Verbindungsstrompfad
- 85: vierter Widerstand

- 90: fünfter Widerstand
- 91: dritte Diode
- 92: vierten Kondensator
- 93: vierte Diode
- 94: fünfter Kondensator
- 95: fünfte Diode
- 96: sechster Kondensator
- 97: sechster Widerstand

- 100: Transistor
- 101: siebter Widerstand
- 102: Halbleitersteuerschalter
- 103: Bipolartransistor

- A: Ansteuersignal
- I: Strom
- I1: erster Strom
- I11: Teilstrom
- I12: Teilstrom
- I2: zweiter Strom
- I21: Teilstrom
- I22: Teilstrom
- I3: dritter Strom
- IV: Versorgungsstrom
- M: Masse
- S: Auswertesignal
- S1: erste Halbwellen
- S2: zweite Halbwellen
- UV: Versorgungsspannung

## Patentansprüche

1. Instrument (26) zur Koagulation und Dissektion von biologischem Gewebe,
mit einem Werkzeug (29), das wenigstens eine Schneidelektrode (38) und wenigstens eine erste Koagulationselektrode (36) und wenigstens eine zweite Koagulationselektrode (37) aufweist,
mit einer Betriebsschaltung (45) aufweisend:
- einen mit der wenigstens einen Schneidelektrode (38) verbundenen Schneidausgang (54), einen mit der wenigstens einen ersten Koagulationselektrode (36) verbundenen ersten Koagulationsausgang (50) und einen mit der wenigstens einen zweiten Koagulationselektrode (37) verbundenen zweiten Koagulationsausgang (51),
- einen Versorgungsanschluss (46), mittels dem eine Versorgungsspannung (UV) oder ein Versorgungsstrom (IV) bereitgestellt wird, und
- einen Auswerteanschluss (24), mittels dem ein Auswertesignal (S) mit ersten Halbwellen (S1) einer Polarität und zweiten Halbwellen (S2) der jeweils anderen Polarität bereitgestellt wird,
- eine mit dem Auswerteanschluss (24) verbundene Auswerteschaltung (70) aufweisend einen manuell betätigbaren ersten Schalter (32) und ein Ansteuerelement (52) zur Erzeugung eines Ansteuersignals (A), wobei die Auswerteschaltung (70) dazu eingerichtet ist, während wenigstens einer der ersten Halbwellen (S1) auszuwerten, ob der erste Schalter (32) betätigt wurde und während wenigstens einer der zweiten Halbwellen (S2) abhängig von der Auswertung das Ansteuersignal (A) zu erzeugen,
- eine mittels eines Ansteuersignals (A) ansteuerbare Umschalteinheit (53), die zwischen einem ersten Schaltzustand und einem zweiten Schaltzustand umschaltbar ist und mit dem Versorgungsanschluss (46) und Schneidausgang (51) verbunden ist.

2. Instrument nach Anspruch 1,
**dadurch gekennzeichnet, dass** die Betriebsschaltung (45) eine Kopplungseinrichtung (71) aufweist, die wenigstens ein Senderbauteil (72) und wenigstens ein galvanisch vom Senderbauteil (72) getrenntes Empfängerbauteil (73) aufweist.

3. Instrument nach Anspruch 2,
**dadurch gekennzeichnet, dass** das Senderbauteil (72) durch das Ansteuerelement (52) gebildet ist und das Empfängerbauteil (73) über eine Lade- und Entladeschaltung (58) mit einem Steuereingang (56) eines ansteuerbaren Halbleiterschalters (55) der Umschalteinheit (53) verbunden ist.

4. Instrument nach einem der vorhergehenden Ansprüche, **dadurch gekennzeichnet, dass** die Umschalteinheit (53) mehrere in Reihe geschaltete Halbleiterschalter (55) aufweist, deren Steuereingänge (56) unmittelbar miteinander verbunden sind.

5. Instrument nach einem der vorhergehenden Ansprüche, **dadurch gekennzeichnet, dass** der erste Schalter (32) und das Ansteuerelement (52) in einem ersten Auswertezweig (75) in Reihe geschaltet sind, wobei der erste Auswertezweig (75) mit dem Auswerteanschluss (24) verbunden ist.

6. Instrument nach Anspruch 5,
**dadurch gekennzeichnet, dass** im ersten Auswertezweig (75) ein Einbahnstrompfad (81) vorhanden ist, in dem das Ansteuerelement (52) angeordnet ist, und dass im Einbahnstrompfad (81) wenigstens ein Bauteil mit Diodenfunktion vorhanden ist, das einen Stromfluss durch den Einbahnstrompfad (81) nur ermöglicht, wenn eine der zweiten Halbwellen (S2) am Auswerteanschluss (24) anliegt.

7. Instrument nach Anspruch 6,
**dadurch gekennzeichnet, dass** das Ansteuerelement (52) als Leuchtdiode ausgeführt ist und/oder im Einbahnstrompfad (81) eine separate Diode (83) in Reihe zum Ansteuerelement (52) geschaltet ist.

8. Instrument nach Anspruch 6 oder 7,
**dadurch gekennzeichnet, dass** der erste Auswertezweig (75) parallel zum Einbahnstrompfad (81) einen Parallelstrompfad (82) aufweist, wobei der erste Schalter (32) in Reihe zum Einbahnstrompfad (81) und zum Parallelstrompfad (82) geschaltet ist.

9. Instrument nach einem der vorhergehenden Ansprüche, **dadurch gekennzeichnet, dass** die Auswerteschaltung (70) einen manuell betätigbaren zweiten Schalter (33) aufweist.

10. Instrument nach Anspruch 9,
**dadurch gekennzeichnet, dass** der zweite Schalter (33) in einem zweiten Auswertezweig (76) angeordnet ist, wobei der zweite Auswertezweig (76) mit dem Auswerteanschluss (24) verbunden ist.

11. Instrument nach Anspruch 9 oder 10,
**dadurch gekennzeichnet, dass** zwischen dem zweiten Schalter (33) und dem Ansteuerelement (52) ein Verbindungsstrompfad (84) vorhanden ist, der bei leitendem zweiten Schalter (33) eine niederohmige Überbrückung des Ansteuerelements (52) bewirkt.

12. Instrument nach einem der vorhergehenden Ansprüche, **dadurch gekennzeichnet, dass** der erste Koagulationsausgang (50) schalterlos mit dem Versorgungseingang (46) verbunden ist.

13. Instrument nach einem der vorhergehenden Ansprüche, **dadurch gekennzeichnet, dass** die Betriebsschaltung (45) eine Transformatorschaltung (60) aufweist, die primärseitig mit dem Versorgungseingang (46) und sekundärseitig mit dem Schneidausgang (54) verbunden ist.

## Claims

1. Instrument (26) for the coagulation and cutting of biological tissue,
with a tool (29) that comprises at least one cutting electrode (38) and at least one first coagulation electrode (36) and at least one second coagulation electrode (37),
with an operating circuit (45) comprising:
- a cutting output (54) connected to the at least one cutting electrode (38), a first coagulation output (50) connected to the at least one first coagulation electrode (36), and a second coagulation output (51) connected to the at least one second coagulation electrode (37),
- a supply connection (46), by means of which a supply voltage (UV) or a supply current (IV) is provided, and
- an evaluation connection (24), by means of which an evaluation signal (S) having first half-waves (S1) of one polarity and second half-waves (S2) of the respectively other polarity is provided,
- an evaluation circuit (70) connected to the evaluation connection (24) comprising a manually actuatable first switch (32) and a triggering element (52) for the generation of a triggering signal (A), wherein the evaluation circuit (70) is configured to evaluate during at least one of the first half-waves (S1) whether the first switch (32) was actuated and to generate, during at least one of the second half-waves (S2), the evaluation signal (A) depending on the evaluation,
- a switching unit (53), which can be actuated by means of a triggering signal (A), which can be switched between a first switching state and a second switching state and which is connected to the supply connection (46) and the cutting output (51) .

2. Instrument according to Claim 1,
**characterized in that** the operating circuit (45) comprises a coupling device (71), which comprises at least one emitter component (72) and at least one receiver component (73) that is galvanically separated from the emitter component (72).

3. Instrument according to Claim 2,
**characterized in that** the emitter component (72) is realized by the triggering element (52), and the receiver component (73) is connected to a control input (56) of a triggerable semiconductor switch (55) of the switching unit (53) via a charging and discharging circuit (58).

4. Instrument according to one of the previous claims, **characterized in that** the switching unit (53) comprises several serially connected semiconductor switches (55), the control inputs (56) of which are directly connected to one another.

5. Instrument according to one of the previous claims, **characterized in that** the first switch (32) and the triggering element (52) are serially connected in a first evaluation branch (75), wherein the first evaluation branch (75) is connected to the evaluation connection (24).

6. Instrument according to Claim 5,
**characterized in that**, a one-way current path (81), in which the triggering element (52) is arranged, is provided in the first evaluation branch (75) and that at least one component with a diode function is provided in the one-way current path (81), that allows a current flow through the one-way current path (81) only when one of the second half-waves (S2) is present at the evaluation connection (24).

7. Instrument according to Claim 6,
**characterized in that** the triggering element (52) is realized as light-emitting diode and/or that a separate diode (83) is connected in series with the triggering element (52) in the one-way current path (81).

8. Instrument according to Claim 6 or 7,
**characterized in that** the first evaluation branch (75) comprises a parallel current path (82) parallel to the one-way current path (81), wherein the first switch (32) is connected in series with the one-way current path (81) and with the parallel current path (82).

9. Instrument according to one of the previous claims, **characterized in that** the evaluation circuit (70) comprises a second switch (33) which can be manually actuated.

10. Instrument according to Claim 9,
**characterized in that** the second switch (33) is arranged in a second evaluation branch (76), wherein the second evaluation branch (76) is connected to the evaluation connection (24).

11. Instrument according to Claim 9 or 10,
**characterized in that**, between the second switch (33) and the triggering element (52) a connecting current path (84) is provided, which effects a low-resistance bridging of the triggering element (52) when the second switch (33) is conductive.

12. Instrument according to one of the previous claims, **characterized in that** the first coagulation output (50) is connected without a switch to the supply input (46).

13. Instrument according to one of the previous claims, **characterized in that** the operating circuit (45) comprises a transformer circuit (60) which is connected to the supply input (46) on its primary side and to the cutting output (54) on its secondary side.

## Revendications

1. Instrument (26) de coagulation et de dissection de tissu biologique,
comprenant un outil (29) qui présente au moins une électrode de coupe (38) et au moins une première électrode de coagulation (36) et au moins une deuxième électrode de coagulation (37),
comprenant un circuit de service (45) présentant :
- une sortie de coupe (54) reliée à l'électrode de coupe (38), au nombre d'au moins une, une première sortie de coagulation (50), reliée à la première électrode de coagulation (36), au nombre d'au moins une, et une deuxième sortie de coagulation (51) reliée à la deuxième électrode de coagulation (37), au nombre d'au moins une,
- une borne d'alimentation (46) à l'aide de laquelle est fournie une tension d'alimentation (UV) ou un courant d'alimentation (IV),
et
- une borne d'évaluation (24) à l'aide de laquelle est fourni un signal d'évaluation (S) avec des premières demi-ondes (S1) d'une polarité et des deuxièmes demi-ondes (S2) respectivement de l'autre polarité,
- un circuit d'évaluation (70) relié à la borne d'évaluation (24) et présentant un premier interrupteur (32) à actionnement manuel et un élément d'activation (52) destiné à produire un signal d'activation (A), le circuit d'évaluation (70) étant conçu pour évaluer, au cours d'au moins une des premières demi-ondes (S1), si le premier interrupteur (32) a été actionné et pour générer le signal d'activation (A) en fonction de l'évaluation, au cours d'au moins une des deuxièmes demi-ondes (S2),
- une unité de commutation (53) qui peut être activée à l'aide d'un signal d'activation (A) et peut être commutée entre un premier état de commutation et un deuxième état de commutation et est reliée à la borne d'alimentation (46) et la sortie de coupe (51).

2. Instrument selon la revendication 1, **caractérisé en ce que** le circuit de service (45) présente un dispositif de couplage (71) qui comporte au moins un composant émetteur (72) et au moins un composant récepteur (73) qui est séparé du point de vue galvanique du composant émetteur (72).

3. Instrument selon la revendication 2, **caractérisé en ce que** le composant émetteur (72) est constitué de l'élément d'activation (52) et le composant récepteur (73) est relié à une entrée de commande (56) d'un commutateur à semi-conducteurs (55) commandé de l'unité de commutation (53), par l'intermédiaire d'un circuit de charge et décharge (58).

4. Instrument selon l'une des revendications précédentes, **caractérisé en ce que** l'unité de commutation (53) présente plusieurs commutateurs à semi-conducteurs (55) montés en série, dont les entrées de commande (56) sont directement reliées les unes aux autres.

5. Instrument selon l'une des revendications précédentes, **caractérisé en ce que** le premier interrupteur (32) et l'élément d'activation (52) sont montés en série dans une première branche d'évaluation (75), la première branche d'évaluation (75) étant reliée à la borne d'évaluation (24).

6. Instrument selon la revendication 5, **caractérisé en ce que** la première branche d'évaluation (75) comporte un chemin de courant à sens unique (81) dans lequel est disposé l'élément d'activation (52), et que le chemin de courant à sens unique (81) comporte au moins un composant à fonction de diode, qui ne permet un écoulement de courant dans le chemin de courant à sens unique (81) que si l'une des deuxièmes demi-ondes (S2) est appliquée à la borne d'évaluation (24).

7. Instrument selon la revendication 6, **caractérisé en ce que** l'élément d'activation (52) est réalisé sous forme de diode électroluminescente et/ou une diode (83) distincte est montée en série avec l'élément d'activation (52).

8. Instrument selon la revendication 6 ou 7, **caractérisé en ce que** la première branche d'évaluation (75) comporte un chemin de courant parallèle (82) qui est parallèle au chemin de courant à sens unique (81), le premier interrupteur (32) étant monté en série avec le chemin de courant à sens unique (81) et le chemin de courant parallèle (82).

9. Instrument selon l'une des revendications précédentes, **caractérisé en ce que** le circuit d'évaluation (70) présente un deuxième interrupteur (33) à commande manuelle.

10. Instrument selon la revendication 9, **caractérisé en ce que** le deuxième interrupteur (33) est disposé dans une deuxième branche d'évaluation (76), la deuxième branche d'évaluation (76) étant reliée à la borne d'évaluation (24).

11. Instrument selon la revendication 9 ou 10, **caractérisé en ce que**, entre le deuxième interrupteur (33) et l'élément d'activation (52), il est prévu un chemin de courant de liaison (84) qui provoque un pontage à faible valeur ohmique de l'élément d'activation (52) lorsque le deuxième interrupteur (33) est conducteur.

12. Instrument selon l'une des revendications précédentes, **caractérisé en ce que** la première sortie de coagulation (50) est reliée sans interrupteur à l'entrée d'alimentation (46).

13. Instrument selon l'une des revendications précédentes, **caractérisé en ce que** le circuit de service (45) présente un circuit transformateur (60) dont le côté primaire est relié à l'entrée d'alimentation (46) et le côté secondaire à la sortie de coupe (54).
